(19) **Europäisches Patentamt / European Patent Office / Office européen des brevets**

(11) **EP 1 605 882 B1**

(12) **EUROPEAN PATENT SPECIFICATION**

(45) Date of publication and mention
of the grant of the patent:
**11.04.2012 Bulletin 2012/15**

(21) Application number: **04702890.7**

(22) Date of filing: **16.01.2004**

(51) Int Cl.:
*A61F 13/15* (2006.01)     *A61L 15/60* (2006.01)
*A61F 13/534* (2006.01)

(86) International application number:
**PCT/SE2004/000048**

(87) International publication number:
**WO 2004/084784 (07.10.2004 Gazette 2004/41)**

(54) **ABSORBENT ARTICLE COMPRISING AN ABSORBENT STRUCTURE**

SAUGFÄHIGER GEGENSTAND MIT ABSORPTIONSSTRUKTUR

ARTICLE ABSORBANT A STRUCTURE ABSORBANTE

(84) Designated Contracting States:
**AT BE BG CH CY CZ DE DK EE ES FI FR GB GR HU IE IT LI LU MC NL PT RO SE SI SK TR**

(30) Priority: **26.03.2003 SE 0300878**

(43) Date of publication of application:
**21.12.2005 Bulletin 2005/51**

(73) Proprietor: **SCA Hygiene Products AB**
**405 03 Göteborg (SE)**

(72) Inventors:
• **GUIDOTTI, Ted**
**S-412 67 Göteborg (SE)**

• **EDWARDSSON, Gunnar**
**S-430 94 Bohus Björkö (SE)**
• **ELIASSON, Malin**
**S-431 46 Mölndal (SE)**

(74) Representative: **Egeröd, Lisbeth**
**Valea AB**
**Lindholmspiren 5**
**417 56 Gothenburg (SE)**

(56) References cited:
**EP-A1- 0 615 736      WO-A1-01/21121**
**WO-A1-91/11162      WO-A1-99/63922**
**US-B1- 6 429 351**

## Description

### Field of the invention

The present invention relates to an absorbent article such as a diaper, an incontinence guard, a sanitary napkin or the like, whereby the article exhibits a liquid permeable upper surface and comprises an absorbent structure, exhibiting a transversal direction and a longitudinal direction, wherein the absorbent structure comprises an acquisition layer and at least one first storage layer, wherein said first storage layer comprises at least 50 percent by weight of a super absorbent material calculated on the total weight of the storage layer.

### Background

Absorbent articles such as diapers, incontinence guards, sanitary napkins, intended for one single use, are usually constructed by an absorbent structure having the capability to acquire large amounts of liquid under a short period of time, having further the ability to distribute the liquid and to store the liquid. This means that the absorbent structure usually comprises several different layers having different properties with respect to each other. Frequently, the absorbent structure at least comprises a liquid acquisition layer and a liquid storage layer. The liquid storage layer often comprises a cellulosic fluff pulp layer mixed with super absorbent material, which are polymers having the ability to absorb water or bodily fluids many times their own weight. The liquid acquisition layer often comprises a porous fibrous layer of synthetic fibres.

Upon usage of such absorbent articles it is desirable that they are thin and discrete to wear, and that they at the same time rapidly can acquire large amounts of liquids discharged under a short period of time and store this liquid in the article.

EP 0 532 002 relates to an absorbent structure, mainly for use in diapers and incontinence guards. The absorbent structure contains at least 30 percent by weight of a super absorbent material and the super absorbent material exhibits some ability to distribute liquid and resist compression during load. The purpose of the described absorbent structure is to obtain a thin structure having a sufficient absorption capability.

EP 0 752 892 relates to an absorbent structure, mainly for use in diapers and incontinence guards. The absorbent structure contains at least 60 percent by weight of a super absorbent material and the super absorbent material exhibits certain properties. The purpose of the described absorbent structure is to obtain a thin structure having a sufficient absorption capability.

However, it has still been shown that it is difficult to obtain sufficient liquid acquiring capacity, liquid distribution capacity and liquid storage capacity, which at the same time are thin and discrete to wear.

### Description of the invention

According to the present invention, an article has been provided which is thin and discrete to wear, and at the same time the article exhibits sufficient liquid acquiring capacity, liquid distribution capacity and liquid storage capacity.

An absorbent article according to the invention is characterised mainly in that the material of the first storage layer in a dry condition, has a density exceeding 0.4 g/cm$^3$, and that said first storage layer exhibits apertures/recesses at least in the crotch portion of the absorbent structure.

Preferably, the material of the first storage layer in a dry condition is even more compressed and exhibits a density exceeding 0.5 g/cm$^3$. According to a further embodiment, the first storage layer exhibits a density exceeding 0.6 g/cm$^3$

According to an embodiment, the material of the first storage layer comprises at least 60 percent by weight of a super absorbent material, calculated on the total weight of the first storage layer in a dry condition. According to another embodiment, the material of the first storage layer comprises at least 70 percent by weight of a super absorbent material, calculated on the total weight of the first storage layer in a dry condition. The material of the first storage layer may also comprise 90 percent by weight or more of the super absorbent material, calculated on the total weight of the first storage layer in a dry condition. An advantage with such a high content of the super absorbent material is that a very thin and discrete article is obtained. *As the content of super absorbent material is high, the amount of the super absorbent material is also relatively high, which in turn leads to that a high absorption capacity and a high liquid storing capacity is achieved.*

According to an embodiment, the apertures/recesses extend through the entire thickness of the first storage layer.

According to an embodiment, the apertures/recesses extend along the longitudinal direction of the absorbent structure. Thus, the apertures/recesses create longitudinal channels in the first storage layer. One advantage with such longitudinal channels is that they direct the liquid transport in a direction towards the end portions of the absorbent structure.

Naturally, it is possible to have other physical designs of the apertures/recesses, for example the apertures/ recesses could be circular or designed in a way that channels are obtained in the transversal direction of the absorbent structure. Using this construction, the first storage layer also functions as a forming element.

According to one embodiment the width of the material between the apertures/recesses, at least in the crotch portion, is maximally 20 mm. A material layer, which firstly comprises super absorbent material at a high content and secondly exhibits a density exceeding 0.4 g/cm$^3$ absorbs relatively slowly. It has also been shown that the liquid transport rate in the planar extension of the storage layer decreases to a large extent after the liquid has been moved about 10 mm. In order to use the total capacity of the entire storage layer, it has thus been shown that the width of the material between the apertures/recesses, at least in the crotch portion, does preferably not exceed 20 mm.

The first storage layer of the absorbent article exhibits a first surface being faced against the liquid permeable surface of the article, and a second surface being faced away from the liquid permeable surface of the article.

According to one embodiment, the acquisition layer lies close to the second surface of the first storage layer, i.e., the surface that in the article is faced away from the user. In the cases where the liquid permeable surface is comprised of a separate liquid permeable top sheet, thus the liquid permeable arranged on either side of the first storage layer. The acquisition layer is preferably a porous layer having the capability to rapidly acquire a large amount of liquid. The first storage layer containing a high percentage of super absorbent material has a better ability to retain liquid than does the acquisition layer. Thus, by placing the storage layer between the liquid permeable top sheet and the acquisition layer, the risk is decreased that the surface being closest to the user becomes wet after a first wetting. In order to obtain a sufficient contact between the layers, it has been shown to be advantageous to join the liquid permeable topsheet to the acquisition layer by means of the hollow spaces being obtained by the apertures/recesses in the first storage layer. Preferably, the joining is thermal. Naturally, it is essential that the thermal joining of the liquid permeable layer and the acquisition layer is sufficient to hold the layers together. However, it has been shown that it is essential that the joining surface does not occupy an area being too large. Upon welding, a liquid impermeable surface is created and if this surface is allowed to be too large, a risk arises that the liquid inlet ability is decreased. In order to eliminate the risk of liquid leakage and at the same time obtain a sufficient joining of the layers, it has, for instance, been shown to be advantageous to use a welding surface, whose width maximally is 1 mm. According to another way of defining the design of the welding surface, it has been shown to be advantageous to design the welding in such a way that the portion of the welded surface, calculated on the entire surface of the top sheet covering the apertures/recesses does not exceed 50 %, more preferably does not exceed 40 %, and most preferably does not exceed 20 %. The welding can, for example, comprise of a plurality of spot welds or weld beads, which are arranged having the same distance to each other or arranged in a particular pattern exhibiting different distances to each other.

According to one embodiment, the acquisition layer lies close to the first surface of the storage layer.

According to one embodiment, the acquisition layer is a super absorbent foam material, for instance a polyacrylate based foam material. A polyacrylate-based foam material is produced by the saturation under pressure using carbon dioxide of a solution, which at least contains monomer, a cross-linking material, an initiator and a tenside in a vessel, during stirring. When the solution is removed from the vessel through a nozzle, the solution is expanded and a foamed structure is achieved. The foamed structure is then locked in that polymerisation and cross-linking are initiated by for instance UV radiation and/or e-beam radiation. Finally, the material is compressed and dried.

An advantage using such a super absorbent foam material is that the material is soft and flexible. Preferably, the foam material exhibits a Gurley stiffness value being lower than 1000 mg. Preferably, the material also exhibits a density in a dry condition exceeding 0.5 g/cm$^3$. Such a super absorbent foam material expands heavily upon contact with water. At the expansion the free volume of the material is increased, leading to that such a super absorbent material can receive a large amount of liquid under a short period of time.

According to another embodiment, the acquisition layer is a fibrous layer including polyacrylate-based particles or a polyacrylate-based coating bonded to the fibrous layer, wherein the polyacrylate-based particles or the polyacrylate-based coating is bonded to the fibrous layer in that acrylic acid monomers are sprayed onto the fibrous layer, whereby the acrylic acid monomer is allowed to polymerise. An example of such a material is a nonwoven material of for instance polyester. Drops of acrylic acid monomers are sprayed onto the nonwoven material, whereby the acrylic acid monomer is allowed to polymerise. The formed polymerised polyacrylic acid particles also function, in addition to as a liquid absorbent material, as a binding agent in that the particles maintain their entire structure in a compressed condition by means of hydrogen bonds between oxygen in the carboxylic groups of the acrylic acid. Upon wetting, the existent hydrogen bonds are broken, whereby the material expands to its uncompressed condition. Thereafter, the material swells further due to the swelling of the super absorbent material upon absorption of liquid. It results in a material being thin and relatively heavily compressed, but when the material thereafter is being wet, the material exhibits a large amount of free volume and high permeability. A further advantage with such an acquisition layer, is that the super absorbent particles bind the liquid not being drained by a storing layer, whereby the risk that the surface being closest to the user becomes wet after a fist wetting, is decreased. The embodiment also covers other ways to bind a super absorbent material to a fibrous structure. In order to further improve such an acquisition layer, it has been shown to be an advantage to corona-treat the acquisition layer. Upon corona treating the layer is treated with plasma, which is a gas being subjected to enough energy to entirely or partly ionise the gas. The contact with the energy-rich gas with the surface of the material, results in that radicals are formed on the surface of the material. Thereafter, different types of functional groups are introduced, such as for example, oxygen-containing functional groups. The advantage using such a corona-treated material is that it exhibits an improved liquid distributing ability compared to a non-corona-treated material.

A further important property of the absorbent structure, is that the liquid transport ratio between the acquisition layer and the first storage layer is such that the first storage layer drains liquid from the acquisition layer. It has been shown that an acquisition layer exhibiting a good ability to be drained on liquid by the acquisition layer, is defined by that at least 50 % of the pores in the material are emptied from liquid at a pressure being lower than 12 cm $H_2O$. The method being used upon measuring the drainage ability is a TRIs porosimeter. The pressure when at least 50 % of the pores in the material are emptied is denoted $PV_{50}$ (cm $H_2O$).

According to a further embodiment, the absorbent structure also comprises a second storage layer. The second storage layer preferably contains a lower amount of super absorbent material calculated on the total weight of the second storage layer. The second storage layer lies for instance close against the liquid permeable back sheet. Further, the second storage layer preferably has a larger extension than the first storage layer in the plane of the article. Thus, the second storage layer functions as an extra security zone, i.e., it absorbs liquid that might be present outside the first storage layer or outside the acquisition layer. It is also possible that the second storage layer entirely or partly encloses the first storage layer. Preferably, the second storage layer is thereby arranged both against the surface of the first storage layer being closest to the user during use of the article, and the surface of the first storage layer which during use of the article is most distantly arranged from the user. The hollow spaces in the first storage layer is essentially free from fibres, however, for the described embodiment using a second storage layer being arranged on each side of the first storage layer, it is possible that single fibres from the second storage layer, entirely or partly, extend through the hollow spaces in the first storage layer. The advantage with such a structure is that the single fibres may direct the liquid transport towards the hollow spaces, and thus facilitate the transport of liquid to the first storage layer. Using this kind of design of the absorbent structure, it is possible to have the first storage layer arranged in the crotch portion of the article and the second storage layer to be arranged both in the crotch portion and in the end portions.

**Brief description of the drawings**

Fig. 1 shows a planar view of an embodiment of an absorbent article according to the invention.

Fig. 2 shows a cross-section of the absorbent article shown in Fig. 1.

Fig. 3 shows a cross-section of an alternative embodiment of an absorbent article according to the invention.

Fig. 4 shows a cross-section of an alternative embodiment of an absorbent article according to the invention.

**Detailed description of the drawings**

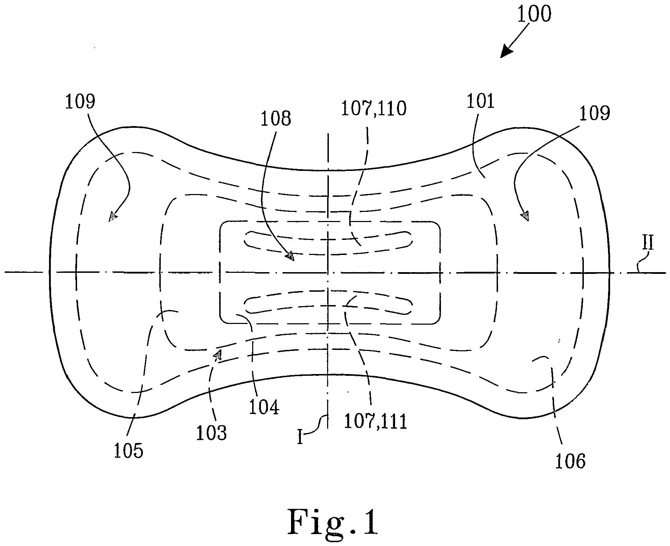

The following description refers to a couple of embodiments of absorbent articles according to the invention, which is thus not limited to the below described embodiments. In Figure 1 a planar view is shown of an absorbent article 100 according to the invention. The absorbent article 100 exhibits a transversal direction, being shown by a transversally extending centre line I, and a longitudinal direction being shown by a longitudinally extending centre line II. Further, the absorbent article 100 exhibits a thickness direction, being directed perpendicularly against the plane. The absorbent article 100 has a liquid permeable top sheet 101, which during use of the article is intended to lie close to the user. Further, the absorbent article 100 has a back sheet 102, which is at least substantially liquid impermeable, and an absorbent structure 103 enclosed between the liquid permeable top sheet and the back sheet. The back sheet material 102 may optionally be a so called vapour permeable breathable material. The absorbent structure exhibits a crotch portion 108 and two end portions 109. The absorbent structure 103 comprises an acquisition layer 104, which is intended to rapidly be able receive a large amount of liquid and a first storage layer 105, which is intended to rapidly be able store a large amount of liquid, and a second storage layer 106. The first storage layer 105 is arranged closest to the liquid permeable top sheet 101, the second storage layer is arranged closest to the substantially liquid impermeable backsheet 102 and the acquisition layer 104 is arranged between the first storage layer 105 and the second storage layer 106. The second storage layer 106 has a longer extension in the plane of the article than the first storage layer 105, but exhibits a lower total absorption capacity. The second storage layer also functions as a form rendering element in such way that it assists in creating and maintaining an absorbent structure being flexible against the body. The first storage layer 105 exhibits two longitudinally arranged apertures/recesses 110, 111. The hollow spaces obtained through the apertures/recesses 110, 111 extend in the longitudinal direction of the article. The distance between the apertures/recesses is preferably maximally 20 mm in the crotch portion. The distance refers to the length of the material between the apertures/recesses 110, 111 in the transversal direction of the article. Close to the end portions 109, the distance between the apertures/recesses is preferably somewhat longer than in the crotch portion. Such a shape is advantageous since both a narrow crotch portion and a shape adapted to the body is obtained.

It is also possible that the apertures/recesses 110, 111 in the first storage layer 105 have other shapes. For example is it possible that the apertures/recesses extend in the transversal direction of the article, whereby transversally arranged channels be obtained. An advantage using this design is that such a storage layer relatively easy may assume a cup shape. Another alternative embodiment is circular apertures/recesses. Naturally, also other shapes of the apertures/recesses are possible.

The storage layers 105, 106 may comprise optional absorbent materials, such as fibrous materials, foam materials, superabsorbent polymers and combinations thereof. According to one embodiment the first storage layer 105 is a fibrous structure comprising at least 50 percent by weight of a super absorbent material calculated on the total weight of the first storage layer 105. Super absorbent materials are polymers having the capability to absorb water or bodily fluids many times their own weight. Conventionally super absorbent materials are polymers such as polyacrylic acid. The super absorbent material is in the shape of powder, flakes, fibres, granules or the like. The super absorbent material may be mixed with the fibre material or may be applied in the form of one or more layers between fibre layers. The super absorbent material is either equally distributed in the first storage layer 105 or distributed in various concentrations in the longitudinal and/or the thickness direction of the first storage layer 105. It is also possible to use a substantially pure layer of super absorbent material in the first storage layer. One example of a suitable super absorbent material is a super absorbent material having the ability to rapidly absorb liquid. A super absorbent material, which can absorb 5 grams of bodily fluids per gram super absorbent material in 10 seconds, is usually defined as a fast super absorbent material. An example of a fast liquid-absorbing super absorbent material is a particulate super absorbent material having a small particle size, i.e., a low particle diameter. Such a particulate super absorbent material usually exhibits an average particle size of between 150 $\mu$m and 400 $\mu$m. It is also possible to use several types of super absorbent material, for example is it possible to use a super absorbent material which absorbs very rapidly in the first storage layer and a conventional super absorbent material absorbing slower in the second storage layer.

Also the second storage layer 106 can according to one embodiment comprise a fibrous structure containing superabsorbent material. The percentage super absorbent material in the second storage layer 106 is preferably lower than the amount of super absorbent material in the first storage layer 105. For example the second storage layer 106 may comprise about 10 percent per weight of a super absorbent material calculated on the total weight of the second storage layer 106. The second storage layer 106 has a longer extension in the plane of the article, but exhibits a lower total absorption capacity.

The bodily fluids, for example urine, penetrates through the liquid permeable top sheet and is then brought via the hollow spaces, i.e., the apertures/recesses in the first storage layer 105, further to the acquisition layer 104. The acquisition layer 104 is then drained on liquid by the first storage layer 105. The acquisition layer 104 may therefore without difficulties receive a second dose of liquid. The first storage layer 105 has the capacity to store several doses of liquid. Since the first storage layer is heavily compressed, it has been shown difficult for the liquid to penetrate the upper part of the structure. A cellulosic fluff pulp mixed with a high content of super absorbent material, whose structure is highly compressed, exhibits a relatively lustry, glossy upper surface, which is difficult to penetrate for the discharged bodily fluid. By providing the apertures/recesses 110, 111 in the highly compressed material, an access to the inner porous structure is created in the first storage layer 105. This facilitates the liquid absorbing capacity of the first storage layer 105.

The acquisition layer 104 is preferably a super absorbent foam material or a fibrous layer having super absorbent particles or a super absorbent coating bound to the fibrous layer. Naturally, the acquisition layer can also constitute of a fibrous layer that do not contain super absorbent material. For example, the acquisition layer may comprise a synthetic fibre layer of for example, polyethylene, polypropylene, polyester, or copolymers thereof. It is also possible that the synthetic fibres are conjugated fibres.

The liquid permeable top sheet is from a non-woven material or an apertured plastic film, or a laminate thereof. Examples of polymers of which the liquid permeable top sheet may be made of is polyethylene, polypropylene, polyester, or copolymers thereof. To enable the liquid permeable top sheet 101 to rapidly let the discharged bodily fluid through, the top sheet is often coated with tensides and/or apertured. Since the first storage layer 105 is highly compressed and exhibits a high density, it is essential that the discharged liquid rapidly (just) reaches the hollow spaces in the first storage layer 105. Therefore, an open liquid permeable top sheet has been shown to be advantageous. An example of an open material is a layer of continuous tow fibres, which are joined in points, lines or spots, in a bonding pattern but are otherwise substantially not connected to each other.

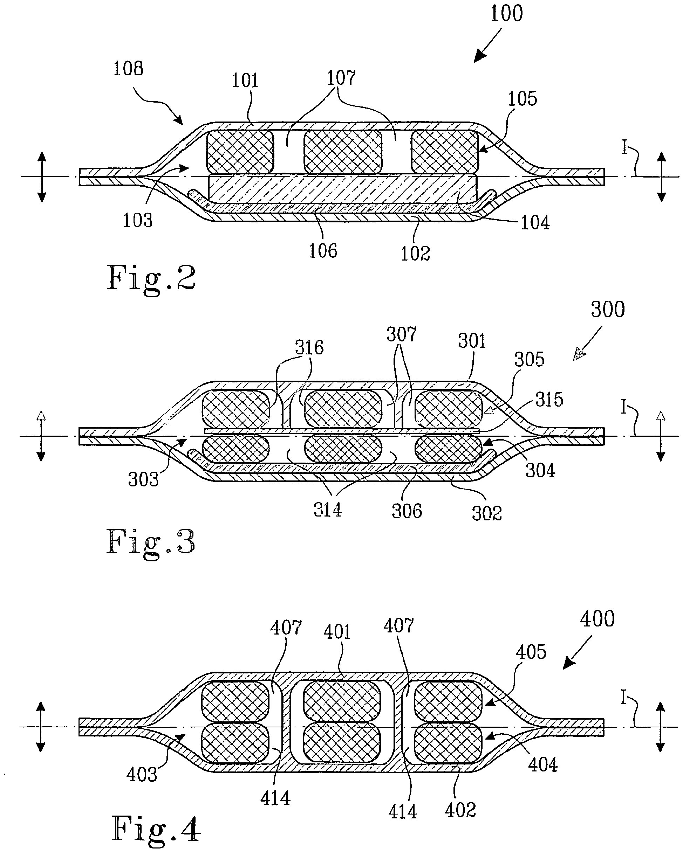

Figure 2 shows a cross-section in the crotch portion 108 of the absorbent article 100 shown in Figure 1. Thus, the absorbent article 100 has a liquid permeable top sheet 101, which during use of the article is intended to lie closest to the user, a back sheet 102, and an absorbent structure 103 enclosed therebetween. The absorbent structure 103 comprises an acquisition layer 104, which is intended to rapidly be able receive a large amount of liquid, a first storage layer 105, which is intended to rapidly be able store a large amount of liquid, and a second storage layer 106 having a longer extension in the plane of the article, but exhibits a lower total absorption capacity. The first storage layer 105 exhibits two longitudinally arranged apertures/recesses/ hollow spaces 107. The distance of the material between the two apertures/recesses is preferably maximally 20 mm in the crotch portion 108. The first storage layer 105 is arranged closest towards the liquid permeable top sheet 101, the second storage layer 106 is arranged closest to the substantially liquid impermeable backsheet 102 and the acquisition layer 104 is arranged between the first storage layer 105 and the second storage layer 106.

Fig. 3 shows a cross-section along a transversally extending centre line I of an alternative embodiment of an absorbent article 300 according to the invention. The absorbent article 300 has a liquid permeable top sheet 301, which during use of the article 300 is intended to lie closest to the user, a back sheet 302, and an absorbent structure 303 enclosed therebetween. The absorbent structure 303 comprises an acquisition layer 304, which is intended to rapidly be able receive a large amount of liquid, a first storage layer 305, which is intended to rapidly be able store a large amount of liquid, and a second storage layer 306 having a longer extension in the plane of the article, but exhibits a lower total absorption capacity than the first storage layer 305. The first storage layer 306 preferably exhibits a lower grammage than the first storage layer 305. The first storage layer 305 is arranged closest to the liquid permeable top sheet 301. The first storage layer 305 exhibits two longitudinally arranged apertures/recesses 107. The first storage layer 305 is a fibre structure containing at least 50 percent by weight of super absorbent material calculated on the total weight of the first storage layer 305.

The second storage layer 306 preferably comprises cellulosic fibres and super absorbent material. The percentage of super absorbent material in the second storage layer 306 is preferably lower than the percentage super absorbent material in the first storage layer 305. The second storage layer 306 functions as a kind of security zone, i.e., the second storage layer 306 will secure that liquid that may appear outside the acquisition layer 304 or the first storage layer 305, does not leak out from the absorbent article, but will instead be absorbed by the second storage layer 306, which in the plane of the article has a longer extension than the acquisition layer 304 or the first storage layer 305. The acquisition layer 304 is arranged between the first storage layer 305 and the second storage layer 306. The acquisition layer 304 comprises three separate strips extending in the longitudinal direction of the article. Between the strips, there is a space being free from the acquisition material, i.e., the hollow space, 314. Preferably the strips in the acquisition layer 304 are comprised of a super absorbent foam material, which in a dry condition is highly compressed. Upon wetting such a super absorbent expands heavily in all directions of the material. The hollow spaces 314 render space for the super absorbent foam material to expand without changing the shape of the absorbent structure. The absorbent structure 303 further comprises a tissue or a substantially hydrophobic nonwoven layer 315, which is arranged between the acquisition layer 304 and the first storage layer 305. The liquid permeable topsheet 301 and the tissue or nonwoven layer 315 are joined to each other. Contact between the layers is obtained in the hollow spaces created in the first storage layer 305 when the apertures/recesses were made. Preferably, it is a thermal joining, but it is also possible to join the liquid permeable top sheet 301 and the tissue or nonwoven layer 315 using an adhesive.

The discharged bodily fluids, for example urine, penetrates through the liquid permeable top sheet 301 and is then brought through the tissue or nonwoven layer 315, whereby it will be rapidly absorbed by the acquisition layer 304. Thereafter, the first storage layer 305 drains the acquisition layer 304 from liquid, whereby the acquisition layer 304 thereafter are prepared to receive the next dose of liquid. The first storage layer has the capacity to store several doses of liquid. In the thickness direction of the first storage layer 305, the walls 316 to the hollow spaces comprise a capillary structure, which is created when the apertures/recesses were made.

Fig. 4 shows a cross-section along a transversally extending centre line I of an alternative embodiment of an absorbent article 400 according to the invention. The absorbent article 400 has a liquid permeable top sheet 401, which during use of the article 400 is intended to lie closest to the user, a substantially liquid impermeable back sheet 402, and an absorbent structure 403 enclosed therebetween. The absorbent structure 403 comprises an acquisition layer 404, which is intended to rapidly be able to receive a large amount of liquid, and a first storage layer 405, which is intended to rapidly be able to store a large amount of liquid. The storage layer 405 is arranged closest to the liquid permeable top sheet 401 and the acquisition layer 404 is arranged closest to the back sheet 402. The first storage layer 405 exhibits two longitudinally arranged apertures/recesses 407. The first storage layer 405 is a fibre structure containing at least 50 percent by weight of super absorbent material calculated on the total weight of the first storage layer 405. The acquisition layer 404 is constituted of three separate strips of material extending in the longitudinal direction of the article. Between the strips, there is a space being free from the acquisition material, i.e., the hollow space, 414. Preferably the strips in the acquisition layer 304 are comprised of a super absorbent foam material, for example a polyacrylate-based foam material. A polyacrylate-based foam material is produced by the saturation under pressure using carbon dioxide of a solution, which at least contains monomer, a cross-linking material, an initiator and a tenside in a vessel during stirring. When the solution is removed from the vessel through a nozzle, the solution is expanded and a foamed structure is achieved. The foamed structure is then locked in that polymerisation and cross-linking are initiated by for instance UV radiation and/or e-beam radiation. Finally, the material is compressed and dried.

Upon wetting such a super absorbent expands heavily in all directions of the material. The hollow spaces 414 render space for the super absorbent foam material to expand without changing the shape of the absorbent structure 403. The liquid permeable topsheet 401 and the back sheet are joined to each other. Contact between the layers is obtained in the hollow spaces created in the storage layer 405 when the apertures/recesses were made. Preferably, it is a thermal joining, but it is also possible to join both layers using an adhesive.

## Example 1

The liquid absorption capacity of the storage layer has been measured for two separate cases. In the first case, liquid is applied to the upper surface of a storage layer not exhibiting any apertures/recesses (sample 1). In the second case, liquid has been applied to the upper surface of a storage layer, which has been cut in the longitudinal direction, in a way that it would constitute a number of separate strips, whereby the strips have been placed with a distance of 1 millimetre from each other (sample 2).

The samples were a mixed structure containing cellulosic fibres and super absorbent particles. The cellulosic fibres were provided from Weyerhauser and are denoted NB 416.

The super absorbent material was a particulate polyacrylate-based super absorbent material from BASF. The super absorbent material is denoted 7160. The samples contained 60 percent by weight of the super absorbent material based on the total weight of the samples.

The area of the test samples was 19.6 cm$^2$. The measurements were performed by putting the samples in a petri dish and subsequently add 10 ml of a sodium chloride solution to the petri dish. The concentration of sodium chloride was 0.9 grams NaCl per litre water. Thereafter, the time for the samples to absorb the liquid was measured.

**Table 1**

| Sample | Total density (g/ cm$^3$) | Absorption rate (sec.) |
|---|---|---|
| Sample 1 | 0.33 | 9 |
| Sample 2 | 0.33 | 8 |
| Sample 1 | 0.45 | 14 |
| Sample 2 | 0.45 | 8 |

The results show that at a high density, it is especially advantageous having apertures/recesses in the material.

## Example 2

The stiffness of the acquisition layer has been measured. The equipment used for the measurement is the "Gurley Precision Instruments Troy" made in New York, USA. The measurements were performed according to the method description "Instructions for Gurley bending resistance/stiffness testers, models 4171 C, D, E". During the measurements, the digital method 4171-D was used.

Tested materials

- Sample 3 is a mixed structure of chemically manufactured cellulosic fluff pulp from Weyerhauser and a particulate polyacrylate-based super absorbent material from BASF. The mixed structure contains 40 percent by weight super absorbent material based on the total weight of the sample.
- Sample 4 is a fibre structure from Weyerhauser. The fibre structure contains 80 percent by weight cross-linked cellulose and 20 percent by weight thermoplastic fibres.
- Sample 5 is a polyester fibre layer having particulate polyacrylate-based super absorbent material bound to the polyester fibre layer. The percentage of super absorbent particles is 60 percent based on the total weight of the sample.
- Sample 6 is a polyacrylate-based super absorbent foam layer. The foam layer is denoted Foam XII and is more closely described in Example 3.
- Sample 7 is a polyacrylate-based super absorbent foam layer. The foam layer is denoted Foam XV and is more closely described in Example 3.

- Sample 8 is a viscose foam material, i.e., a foam material from regenerated cellulose.

The measurements were performed at three different densities for all samples. The samples were measured at the densities 0.50 g/cm³, 0.71 g/cm³, and 0.91 g/cm³. The samples were compressed to the given density and after 10-30 seconds, the samples were placed in the test equipment. In the table below the results from the measurements are shown. The density is given in g/ cm3 and the stiffness in milligrams.

**Table 2**

**Gurley stiffness (milligrams)**

| Sample | 0.50 g/ cm³ | 0.71 g/ cm³ | 0.91 g/ cm³ |
|---|---|---|---|
| Sample 3 | 1343 | 2364 | 2359 |
| Sample 4 | 3437 | 3623 | 3823 |
| Sample 5 | 5090 | 5894 | 6088 |
| Sample 6 | 105 | 69 | 56 |
| Sample 7 | 274 | 123 | 141 |
| Sample 8 | 9246 | 5690 | 5023 |

The results clearly show that the super absorbent foam layers, i.e., sample 6 and sample 7, are significantly softer and more flexible than the other test samples.

**Example 3**

The pores in the acquisition layer made of polyacrylate-based foam materials have been characterised by means of a Liquid Porosimeter equipment from Textile Research Institute, Princeton, USA. The function of the equipment is described in detail in Miller, B. and Tyomkin, I. in Journal of Colloid and Interface Science, 162, 163-170 (1994).

The tested materials are two kinds of polyacrylate-based foam materials, Foam XII and Foam XV, respectively. Foam XII has been made according to the following:

To a beaker the following is added:

- 348.5 grams of acrylic acid (4.84 moles)
- 135.5 grams of a sodium acrylate solution containing 37.3 percent per weight (0.54 moles)
- 28.0 grams of polyethylene glycol diacrylate from polyethylene glycol having a molecular weight of 400.
- 21.3 grams of a aqueous solution 15 percent per weight containing ethylene oxide and linear $C_{16}$-$C_{18}$ fatty alcohol (molar ratio 80:1)
- 65.7 grams of water.

The ingredients were mixed and thereafter, the solution was cooled to a temperature lower than 16 °C. The solution was the poured into a closed container, whereby the solution was saturated with carbon dioxide at a pressure of 12 bars for 25 minutes. Using the same pressure, 26.7 grams of an aqueous solution containing 3 percent by weight of 2,2'-azobis(2-amidinopropane) dihydrochloride was added. This was mixed to a homogenous solution. The solution was then allowed to rest in five minutes. The saturated solution was compressed from a container using a nozzle having an opening being 1 mm at a pressure being 12 bars. The resulting monomeric foam was placed on a glass plate (DIN-A3). An additional glass plate was then placed on top of the monomeric foam. Then, the foam was polymerised using a UV/VIS lamp, a UV1000 lamp from Höhnle. The foam was illuminated using the lamp both from underneath and from above. The illumination and thereby also the polymerisation were allowed to proceed for 4 minutes. Foam XV was made in the same way. The difference between Foam XII and Foam XV was that more cross-linking agent (i.e., polyethylene glycol diacrylate) was used for making Foam XV. 40.0 grams of polyethylene glycol diacrylate instead of 28.0 grams was added for making Foam XV.

PV50 is the pressure when 50 % of the drainable pores have been emptied. The super absorbent foam materials that are shown to be especially advantageous, exhibit a $PV_{50}$ value being lower than 12 cm $H_2O$. The $PV_{50}$ value is obtained by measuring the amount of liquid as a function of the pressure in the chamber in a receding measurement and register when 50 % of the drainable pores have been emptied. Upon a receding measurement, the amount of liquid is measured, being emptied from a porous material at a certain pressure in the chamber. At the measurement excess liquid is delivered to the sample. The sample is allowed to absorb this liquid. Then the sample is placed in the chamber on a membrane and a porous plate. A mechanical load is applied. Thereafter the chamber is closed and the air pressure inside the chamber is raised successively in steps by means of a computer-controlled pressure maintaining system, whereby the liquid leaves the sample through a membrane having small pores. The weight of the squeezed liquid is registered using a beam balance.

The amount of liquid present in the sample upon full saturation when it is in the chamber is estimated, $M_o(g)$. The liquid remaining in the sample when the pressure in the chamber exceeds 50 cm $H_2O$ is considered to be a difficultly drainable liquid. This amount of liquid is estimated $M_D(g)$. The pressure at which 50 % of the drainable liquid has been drained out of the samples is calculated according to the following:

$$M_{50\%} = 0.5\,(M_o - M_D) + M_D$$

From the protocol from receding measurements, the pressure at which the chamber had when the sample contained the amount $M_{50\%}$, can be estimated. This pressure is the $PV_{50}$ value. $PV_{50}$ should be lower than 12 cm $H_2O$, more preferably lower than 8 cm $H_2O$, and most preferred lower than 6 cm $H_2O$. For the material to be able to keep the liquid in a satisfactory manner, the $PV_{50}$ value should not be lower than 2 cm $H_2O$, and preferably not lower than lower than 4 cm $H_2O$.

A more detailed description of how the measurements were performed follows below.

Before the measurement the samples were kept in sealed plastic bags in order to avoid absorption of moisture from the air. The dry sample was weighed. Thereafter, the sample was placed in the test chamber on membranes (Millipore 0.22 $\mu$m cat. No. GSWP 09000), whereby the sample was allowed to swell in excess liquid during 30 minutes. The size of the sample after swelling was 10-25 cm$^2$.

At this measurement synthetic urine was used. The ion concentration in the liquid was 0.135 M sodium, 0.086 M potassium, 0.0035 M magnesium, 0.002 M calcium, 0.19 M chloride, 0.0055 M sulphate, and 0.031 phosphate. Additionally, the liquid contained 0.3 M urea and 1 ppm w/w Triton TX-100 (Calbiochem-648462). The liquid was made in such a way that no salts were precipitated.

A load covering the whole sample surface was placed on the sample during the swelling and the measurement. To avoid measuring pores between the sample surface and the load and to maintain an equal load distribution over the whole sample surface, non-absorbent polyurethane foam was placed between the sample and the applied load. The total load put on the swelled sample was 0.3 kPa.

The equilibrium velocity, i.e., the velocity when the weight change at the selected air pressure had decreased to an insignificant level, was upon measuring 5 mg/min and the measure time during which the weight change was recorded was 30 seconds. The measurements were made at the following applied air pressures measured in cm $H_2O$: 1.1-1.2-1.4-1.7-2.0-2.2-2.4-2.7-3.1-3.5-4.1-4.4-4.7-5.1-5.6-6.1-6.8-7.7-8.7-10.2-11.1-12.2-13.6-15.3-17.5-20.4-24.5-30.6-40.8-49.0-61.2.

To record the remaining liquid, the sample was weighed directly after each terminated measurement. In addition to the measurement on samples one blank control run was performed. At the control run, only foam and load was placed in the test chamber. The measurement was performed the same way and using the same conditions as for the sample measurements. The control run is then subtracted from the sample run before continued processing of raw data.

**Table 3**

| Sample | $PV_{50}$ |
|---|---|
| Foam XII | 5.3 cm $H_2O$ |
| Foam XV | 17 cm $H_2O$ |

**Claims**

1. Absorbent article (100; 300; 400) such as a diaper, an incontinence guard, a sanitary napkin or the like, whereby the article (100; 300; 400) exhibits a liquid permeable upper surface (101) and comprises an absorbent structure (103; 303; 403), which In the longitudinal direction (II) exhibits a crotch portion (108) and two end portions (109), wherein the absorbent structure (103; 303; 403) comprises an acquisition layer (104; 304; 404) and at least one first storage layer (105; 305; 405), wherein said first storage layer (105; 305; 405) comprises at least 50 percent by weight of a super absorbent material calculated on the total weight of the first storage layer (105; 305; 405), *characterised in,* **that** the material of the first storage layer (105; 305; 405) in a dry condition has a density exceeding 0.4 g/cm$^3$, and that said first storage layer (105; 305; 405) at least in the crotch portion (108) of the absorbent structure (103) exhibits apertures/recesses (110, 111; 307; 407).

2. Absorbent article according to claim 1, ***characterised in,* that** the material of the first storage layer (105; 305; 405) in a dry condition, exhibits a density exceeding 0.5 g/cm$^3$.

3. Absorbent article according to claim 1 or claim 2, ***characterised in,* that** the first storage layer (105; 305; 405) comprises at least 70 percent by weight of a super absorbent material calculated on the total weight of the first storage layer (105; 305; 405).

4. Absorbent article according to any of the preceding claims, ***characterised in,* that** the apertures/recesses (110, 111; 307; 407) extend through the entire thickness of the first storage layer (105; 305; 405).

5. Absorbent article according to any of the preceding claims, ***characterised in,* that** the apertures/recesses (110, 111; 307; 407) extend along the longitudinal direction of the absorbent structure (103; 303; 403), wherein the apertures/recesses (110, 111; 307; 407) comprise longitudinal channels.

6. Absorbent article according to any of the preceding claims, ***characterised in,* that** the material between the apertures/recesses (110, 111; 307; 407), at least in the crotch portion (108) of the first storage layer (105; 305; 405), exhibits a width being maximally 20 mm.

7. Absorbent article according to any of the preceding claims, wherein the first storage layer (105; 305; 405) exhibits a first surface being faced against the liquid permeable surface of the article, and a second surface being faced away from the liquid permeable surface of the article, ***characterised in,* that** the acquisition layer (104; 304; 404) lies close to the first surface of the storage layer (105; 305; 405).

8. Absorbent article according to any of the claims 1-6, wherein the first storage layer (105; 305; 405) exhibits a first surface being faced against the liquid permeable surface of the article, and a second surface being faced away from the liquid permeable surface of the article, ***characterised in,* that** the acquisition layer (104; 304; 404) lies close to the second surface of the storage layer (105; 305; 405).

9. Absorbent article according to claim 8, ***characterised in,* that** it comprises a liquid permeable top sheet (101), wherein the liquid permeable top sheet (101) and the acquisition layer (104; 304; 404) are thermally joined in a hollow space in the first storage layer (105; 305; 405) created by said apertures/recesses (110, 111; 307; 407).

10. Absorbent article according to any of the claims 1-9, ***characterised in,* that** the acquisition layer (104; 304; 404) is a polyacrylate based super absorbent foam material.

11. Absorbent article according to any of the preceding claims, ***characterised in,* that** said foam material exhibits a Gurley stiffness value being lower than 1000 mg and a density in a dry condition exceeding 0.5 g/cm$^3$.

12. Absorbent article according to any of the claims 1-9, ***characterised in,* that** the acquisition layer (104; 304; 404) is a fibrous layer including polyacrylate-based particles or a polyacrylate-based coating bonded to the fibrous layer, wherein the polyacrylate-based particles or the polyacrylate-based coating is bonded to the fibrous layer **in that** acrylic acid monomers are sprayed onto the fibrous layer whereby the acrylic add monomer is allowed to polymerise.

13. Absorbent article according to any of the preceding claims, ***characterised in,* that** the acquisition layer (104) is corona treated.

14. Absorbent article according to any of the preceding claim, ***characterised in,* that** the absorbent structure (103) also includes a second storage layer (106) containing a lower amount of super absorbent material calculated on the total weight of the storage layer.

**Patentansprüche**

1. Absorptionsartikel (100; 300; 400), wie zum Beispiel eine Windel, ein Inkontinenzschutz, eine Binde oder dergleichen, wobei der Artikel (100; 300; 400) eine flüssigkeitsdurchlässige obere Oberfläche (101) und eine Absorptionsstruktur (103; 303; 403) aufweist, die in der Längsrichtung (II) einen Schrittabschnitt (108) und zwei Endabschnitte (109) aufweist, wobei die Absorptionsstruktur (103; 303; 403) eine Aufnahmelage (104; 304; 404) und zumindest eine erste Speicherlage (105; 305; 405) aufweist, wobei die erste Speicherlage (105; 305; 405) zumindest 50 Gew-% eines superabsorbierenden Materials aufweist, berechnet auf das Gesamtgewicht der ersten Speicherlage (105; 305; 405), **dadurch gekennzeichnet, dass** das Material der ersten Speicherlage (105; 305; 405) in einem trockenen Zustand eine Dichte aufweist, die 0,4 g/cm$^3$ übersteigt, und dass die erste Speicherlage (105; 305; 405) zumindest in dem Schrittabschnitt (108) der Absorptionsstruktur (103) Öffnungen/Aufnahmen (110, 111; 307; 407) aufweist.

2. Absorptionsartikel nach Anspruch 1, **dadurch gekennzeichnet, dass** das Material der ersten Speicherlage (105; 305; 405) in einem trockenen Zustand eine Dichte aufweist, die 0,5 g/cm$^3$ übersteigt.

3. Absorptionsartikel nach Anspruch 1 oder Anspruch 2, **dadurch gekennzeichnet, dass** die erste Speicherlage (105; 305; 405) zumindest 70 Gew-% eines superabsorbierenden Materials aufweist, berechnet auf das Gesamtgewicht der ersten Speicherlage (105; 305; 405) .

4. Absorptionsartikel nach einem der vorhergehenden Ansprüche, **dadurch gekennzeichnet, dass** sich die Öffnungen/Aufnahmen (110, 111; 307; 407) durch die gesamte Dicke der ersten Speicherlage (105; 305; 405) erstrecken.

5. Absorptionsartikel nach einem der vorhergehenden Ansprüche, **dadurch gekennzeichnet, dass** die Öffnungen/Aufnahmen (110, 111; 307; 407) sich entlang der Längsrichtung der Absorptionsstruktur (103; 303; 403) erstrecken, wobei die Öffnungen/Aufnahmen (110, 111; 307; 407) Längskanäle aufweisen.

6. Absorptionsartikel nach einem der vorhergehenden Ansprüche, **dadurch gekennzeichnet, dass** das Material zwischen den Öffnungen/Aufnahmen (110, 111; 307; 407) zumindest in dem Schrittabschnitt (108) der ersten Speicherlage (105; 305; 405) eine Breite aufweisen, die maximal 20 mm beträgt.

7. Absorptionsartikel nach einem der vorgehenden Ansprüche, wobei die erste Speicherlage (105; 305; 405) eine erste Oberfläche, die gegen die flüssigkeitsdurchlässige Oberfläche des Artikels gewandt ist, und eine zweite Oberfläche, die von der flüssigkeitsdurchlässigen Oberfläche des Artikels weg gewandt ist, aufweist, **dadurch gekennzeichnet, dass** die Aufnahmelage (104; 304; 404) nahe an der ersten Oberfläche der Speicherlage (105; 305; 405) liegt.

8. Absorptionsartikel nach einem der Ansprüche 1 bis 6, wobei die erste Speicherlage (105; 305; 405) eine erste Oberfläche aufweist, die gegen die flüssigkeitsdurchlässige Oberfläche des Artikels gewandt ist, und eine zweite Oberfläche, die von der flüssigkeitsdurchlässigen Oberfläche des Artikels weg gewandt ist, **dadurch gekennzeichnet, dass** die Aufnahmelage (104; 304; 404) nahe an der zweiten Oberfläche der Speicherlage (105; 305; 405) liegt.

9. Absorptionsartikel nach Anspruch 8, **dadurch gekennzeichnet, dass** er eine flüssigkeitsdurchlässige Oberschicht (101) aufweist, wobei die flüssigkeitsdurchlässige Oberschicht (101) und die Aufnahmelage (104; 304; 404) in einem hohlen Raum in der ersten Speicherlage (105, 305, 405), der durch die Öffnungen/Aufnahmen (110, 111; 307; 407) gebildet ist, thermisch verbunden sind.

10. Absorptionsartikel nach einem der Ansprüche 1 bis 9, **dadurch gekennzeichnet, dass** die Aufnahmelage (104; 304; 404) ein Polyacrylat basiertes superabsorbierendes Schaummaterial ist.

11. Absorptionsartikel nach einem der vorhergehenden Ansprüche, **dadurch gekennzeichnet, dass** das Schaummaterial einen Gurley Steifheitswert von weniger als 1000 mg und eine Dichte in einem trockenen Zustand von mehr als 0,5 g/cm$^3$ aufweist.

12. Absorptionsartikel nach einem der Ansprüche 1 bis 9, **dadurch gekennzeichnet, dass** die Aufnahmelage (104; 304; 404) eine Faserlage mit Polyacrylat basierten Partikeln oder einer Polyacrylat basierten Beschichtung ist, die an die Faserlage gebunden sind/ist, wobei die Polyacrylat basierten Partikel oder die Polyacrylat basierte Beschichtung an die Faserlage derart gebunden sind/ist, dass Acrylsäuremonomere auf die Faserlage gesprüht sind, wobei das Acrylsäuremonomer polymerisieren kann.

13. Absorptionsartikel nach einem der vorhergehenden Ansprüche, **dadurch gekennzeichnet, dass** die Aufnahmelage (104) coronabehandelt ist.

14. Absorptionsartikel nach einem der vorhergehenden Ansprüche, **dadurch gekennzeichnet, dass** die Absorptionsstruktur (103) auch eine zweite Speicherlage (106) aufweist, die eine kleinere Menge von superabsorbierendem Material aufweist, berechnet auf das Gesamtgewicht der Speicherlage.

**Revendications**

1. Article absorbant (100 ; 300 ; 400) tel qu'une couche, une protection contre l'incontinence, une serviette hygiénique ou article similaire, dans lequel l'article (100 ; 300 ; 400) présente une surface supérieure perméable aux liquides (101) et comprend une structure absorbante (103 ; 303 ; 403), qui présente dans la direction longitudinale (II) une partie d'entrejambe (108) et deux parties d'extrémité (109), dans lequel la structure absorbante (103 ; 303 ; 403) comprend une couche d'acquisition (104 ; 304 ; 404) et au moins une première couche de stockage (105 ; 305 ; 405), dans lequel ladite première couche de stockage (105 ; 305 ; 405) comprend au moins 50 pour cent en poids d'un matériau superabsorbant, calculé sur le poids total de la première couche de stockage (105 ; 305 ; 405), **caractérisé en ce que** le matériau de la première couche de stockage (105 ; 305 ; 405), dans un état sec, a une masse volumique supérieure à 0,4 g/cm$^3$, et **en ce que** ladite première couche de stockage (105 ; 305 ; 405), au moins dans la partie d'entrejambe (108) de la structure absorbante (103), présente des ouvertures ou évidements (110, 111 ; 307 ; 407).

2. Article absorbant selon la revendication 1, **caractérisé en ce que** le matériau de la première couche de stockage (105 ; 305 ; 405), dans un état sec, présente une masse volumique supérieure à 0,5 g/cm$^3$.

3. Article absorbant selon la revendication 1 ou 2, **caractérisé en ce que** la première couche de stockage (105 ; 305 ; 405) comprend au moins 70 pour cent en poids d'un matériau superabsorbant, calculé sur le poids total de la première couche de stockage (105 ; 305 ; 405).

4. Article absorbant selon l'une quelconque des revendications précédentes, **caractérisé en ce que** les ouvertures ou évidements (110, 111 ; 307 ; 407) s'étendent à travers toute l'épaisseur de la première couche de stockage (105 ; 305 ; 405).

5. Article absorbant selon l'une quelconque des revendications précédentes, **caractérisé en ce que** les ouvertures ou évidements (110, 111 ; 307 ; 407) s'étendent le long de la direction longitudinale de la structure absorbante (103 ; 303 ; 403), dans lequel les ouvertures ou évidements (110, 111 ; 307 ; 407) comprennent des canaux longitudinaux.

6. Article absorbant selon l'une quelconque des revendications précédentes, **caractérisé en ce que** le matériau présent entre les ouvertures ou évidements (110, 111 ; 307 ; 407), au moins dans la partie d'entrejambe (108) de la première couche de stockage (105 ; 305 ; 405), a une largeur inférieure ou égale à 20 mm.

7. Article absorbant selon l'une quelconque des revendications précédentes, dans lequel la première couche de stockage (105 ; 305 ; 405) présente une première surface placée contre la surface perméable aux liquides de l'article, et une deuxième surface tournée à l'écart de la surface perméable aux liquides de l'article, **caractérisé en ce que** la couche d'acquisition (104 ; 304 ; 404) se trouve près de la première surface de la couche de stockage (105 ; 305 ; 405).

8. Article absorbant selon l'une quelconque des revendications 1 à 6, dans lequel la première couche de stockage (105 ; 305 ; 405) présente une première surface placée contre la surface perméable aux liquides de l'article, et une deuxième surface tournée à l'écart de la surface perméable aux liquides de l'article, **caractérisé en ce que** la couche d'acquisition (104 ; 304 ; 404) se trouve près de la deuxième surface de la couche de stockage (105 ; 305 ; 405).

9. Article absorbant selon la revendication 8, **caractérisé en ce qu'**il comprend une feuille supérieure perméable aux liquides (101), dans lequel la feuille supérieure perméable aux liquides (101) et la couche d'acquisition (104 ; 304 ; 404) sont liées thermiquement dans un espace creux dans la première couche de stockage (105 ; 305 ; 405) créé par lesdites ouvertures ou évidements (110, 111 ; 307 ; 407).

10. Article absorbant selon l'une quelconque des revendications 1 à 9, **caractérisé en ce que** la couche d'acquisition (104 ; 304 ; 404) est faite d'un matériau de type mousse superabsorbante à base de polyacrylate.

**11.** Article absorbant selon l'une quelconque des revendications précédentes, **caractérisé en ce que** ledit matériau de type mousse présente une valeur de rigidité Gurley inférieure à 1000 mg et une masse volumique à l'état sec supérieure à 0,5 g/cm$^3$.

**12.** Article absorbant selon l'une quelconque des revendications 1 à 9, **caractérisé en ce que** la couche d'acquisition (104 ; 304 ; 404) est une couche fibreuse comprenant des particules à base de polyacrylate ou un revêtement à base de polyacrylate collé à la couche fibreuse, dans lequel les particules à base de polyacrylate ou le revêtement à base de polyacrylate est collé à la couche fibreuse du fait d'une pulvérisation de monomères d'acide acrylique sur la couche fibreuse, après laquelle on laisse polymériser le monomère d'acide acrylique.

**13.** Article absorbant selon l'une quelconque des revendications précédentes, **caractérisé en ce que** l'on fait subir à la couche d'acquisition (104) un traitement corona.

**14.** Article absorbant selon l'une quelconque des revendications précédentes, **caractérisé en ce que** la structure absorbante (103) comprend aussi une deuxième couche de stockage (106) contenant une quantité inférieure de matériau superabsorbant, calculée sur le poids total de la couche de stockage.

Fig.1

Fig.2

Fig.3

Fig.4

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0532002 A

- EP 0752892 A

**Non-patent literature cited in the description**

- **MILLER, B. ; TYOMKIN, I.** *Journal of Colloid and Interface Science,* 1994, vol. 162, 163-170